Europäisches Patentamt

**European Patent Office**

**Office européen des brevets**

(19)

(11) Publication number: **0 047 641**
**B2**

(12)

# NEW EUROPEAN PATENT SPECIFICATION

(45) Date of publication of the new patent specification:
23.06.89

(51) Int. Cl.⁴: **C 12 P 7/06**

(21) Application number: **81304044.1**

(22) Date of filing: **04.09.81**

(54) Ethanol production by high performance bacterial fermentation.

(30) Priority: **05.09.80 US 184508**
**16.12.80 US 217066**

(43) Date of publication of application:
**17.03.82 Bulletin 82/11**

(45) Publication of the grant of the patent:
**25.04.84 Bulletin 84/17**

(45) Mention of the opposition decision:
**23.06.89 Bulletin 89/34**

(84) Designated Contracting States:
**CH DE FR GB LI NL SE**

(56) References cited:
**WO-A-81/01012**
**GB-A-2 055 121**
**US-A-2 169 244**

**CHEMICAL ABSTRACTS, volume 93, August 18, 1980, abstract 68645q COLUMBUS OHIO (US) & Biotechnol. Lett. 1980, 2(5), 241-6 L. HAEGGSTROEM et al. "Calcium alginate immobilized cells of Clostridium acetobutylicum for solvent production"**
**MICROBIOLOGY ABSTRACTS, volume 14, no. 10, 1979 LONDON (GB) P.L ROGERS et al. "Kinetics of alcohol production by Zymomonas mobilis at high sugar concentrations", page 14, (7468-A14)**
**Abstract VIth International Fermentation Symposium and Vth International Symposium on Yeast, 20 to 25 July 1980, London, Ontario, Canada, Abstract F-9.3.8 (P) Ethanol production by Zymomonas mobilis (T. Chase, Jr., D.E. Eveleigh,**

(73) Proprietor: **GEORGE WESTON LIMITED, 22 St. Clair Avenue East, Toronto Ontario (CA)**

(72) Inventor: **Lawford, Hugh Gibson, 2383 Edenhurst Drive, Mississauga Ontario (CA)**

(74) Representative: **Ford, Michael Frederick, MEWBURN ELLIS & CO. 2/3 Cursitor Street, London EC4A 1BQ (GB)**

(56) References cited: (continuation)
**R. Pincus and B.S. Montenecourt)**
**Bioconversion and Biochemical Engineering, Vol. 2, pp. 359-369**
**P.L. Rogers, Kye Joon Lee and D.E. Tribe, Biotechnology Letters, 1, pp. 165-170, 1979**
**H.J. Rehm, Industrielle Mikrobiologie, 1980, Zweite Auflage, Kapitel 10, pp. 134-140**
**T.P. Lyons, A Step to Energy Independence (a Textbook for Fuel Alcohol Fermentation), Alltech Technical Publications, Kentucky, 1981, pp 92-93**

EP 0 047 641 B2

## Description

The present invention relates to a process for the production of ethanol by fermentation. In particular it relates to the production of ethanol by a process of bacterial fermentation, in which there is improved fermentation efficiency and product yield as compared to prior art methods.

The traditional process of fermentation is carried out in a conventional batch operation utilizing yeast as the fermenting organism. To increase the efficiency a variation of the batch operation occasionally includes recycling of the yeast cells by systems such as sedimentation, centrifugation, or ultrafiltration. Normally this batch operation is conducted in two stages. The first stage involves propagation of the yeast and is referred to as the growth stage. The second stage involves the anaerobic process of ethanol production which is accompanied by a depletion of the oxygen. Further propagation of the yeast occurs during the anaerobic process of ethanol production.

Typically, a yeast inoculum is prepared in stage one. The requirements for maximum yeast reproduction are adequate amounts of carbon, nitrogen, minerals and oxygen, a pH in the range of 3.5 to 4.5, and a temperature in the range of 29 - 35°C. Aerobic growth conditions define a system for more efficient production of yeast but under which no ethanol is produced.

Stage two is the fermentation stage where the alcohol is actually produced by the yeast from the fermentable sugars. The yeast inoculum produced in stage (i) is used to seed a large fermenter previously filled with the substrate, which may be molasses, corn, etc., adjusted to the appropriate pH, temperature and sugar concentration. The inoculation rate can be 5 to 10 million cells per ml and during the fermentation the viable cell count can increase to 150 - 200 million cells per ml. Heat produced is controlled through the use of cooling coils. At these yeast levels, a final ethanol concentration of about 9 to 11 % (v/v) can be obtained in 30 to 70 hours with batch fermentation. Increasing the yeast content, as is the case with cell recycle, can considerably reduce the time required for completion of the fermentation. For example, with a cell density of 800 to 1000 million cells per ml, it is possible to reduce the fermentation time to 4 to 10 hours.

It is recognized that, although conditions of strict anaerobiosis promote maximum production of ethanol, growth of yeast is suppressed. Inherent in this fermentation process is the coupling of growth with the rate of alcohol production. Consequently, in order to optimize ethanol production, either the degree of aeration must be finely controlled, or the medium must be supplied with growth-promoting supplements (for example, ergosterol or unsaturated fatty acids such as oleic acid).

The present invention describes a process for the production of ethanol by bacterial rather than yeast fermentation. The process utilizes the bacterium Zymomonas as the fermenting organism. A number of different strains of Zymomonas mobilis may be used; for example ATCC 29191, ATCC 10988, etc. The process consists of two stages, an initial stage for the production of biomass, and a second stage for the production of ethanol. The growth stage in this process occurs anaerobically and is accompanied by the production of ethanol. The fermentation stage also occurs under anaerobic conditions. However, in the bacterial process according to the present invention the production of ethanol is essentially uncoupled from growth (i.e. production of biomass). Thus, during the production of ethanol from such a "resting culture", only a small proportion of the substrate is converted to biomass and ethanol production is maximized. It follows that production of ethanol in the absence of growth with the bacterial process can be achieved simply by the addition of fermentable sugar and this system thereby is capable of yielding high alcohol levels. Production of ethanol using Zymomonas is disclosed by P.L. Rogers, et al in Biotechnilogy Letters 1 165 - 70 (1979).

Accordingly, the present invention comprises a process for producing ethanol by fermentation, which comprises culturing a microorganism capable of producing ethanol and which is a number of the genus Zymomonas two stages comprising

(i) producing a bacterial cell suspension together with ethanol in an ethanol concentration range that does not substantially inhibit production of the microbial cells in a medium containing a source of nitrogen and a source of carbon and

(ii) producing ethanol, in the absence of substantial bacterial cell production, by the addition of fermentable sugar to the bacterial cell suspension produced in (i), wherein the sugar concentration in stage (ii) does not exceed 6 % w/v and wherein either

a) the bacterial cells are collected and resuspended in further medium between stages (i) and (ii) or

b) the two stages of the process are conducted in two fermenters with the bacterial cells produced by continuous culture in the first fermenter being continuously transferred to the second of the fermenters for Stage (ii).

## Stage (i)

Growth of Zymomonas mobilis is conducted in an aqueous nutrient medium. The medium may be either a natural nutrient medium, a synthetic culture medium or a semi-synthetic culture medium as long as a suitable carbohydrate source and the essential nutrients for the growth of the microorganism are present. Although the growth stage in the process is normally used to generate sufficient quantities of biomass for the operation of the fermentation stage, the biomass may also be recycled with concomitant reduction in the essential nutrients required by the process.

In general, conditions which may be used to promote growth of Z. mobilis are: adequate carbon supply, adequate nitrogen supply, appropriate organic growth factors, adequate mineral supply, essentially anaerobic conditions, agitation of the culture, temperature in the range of 20 - 40°C, and pH in the range of 4 to 8.

As carbon source for both the growth and the fermentation stages of the process various carbohydrates may be used. The carbohydrates include, for example, sugars such as glucose, fructose, sucrose; molasses; starch hydrolysate; cellulose hydrolysate; etc. These substances may be used either singly or in mixtures of two or more.

As a nitrogen source, various kinds of inorganic or organic salts or compounds, for example ammonium salts such as ammonium chloride, ammonium sulfate, etc., or natural substances containing nitrogen, such as yeast extract, casein hydrolysate, corn steep liquor etc., or amino acids such as glutamic acid may be employed. These substances may also be used either singly or in combinations of two or more.

Inorganic compounds which may be added to the culture medium include magnesium sulfate, potassium monohydrogen phosphate, potassium dihydrogen phosphate, sodium chloride, magnesium sulfate, calcium chloride, iron chloride, magnesium chloride, zinc sulfate, cobalt chloride, copper chloride, borates, molybdates, etc.

Organic compounds which may be desirable for the operation of the process include, for example, vitamins such as biotin, calcium pantothenate, and the like, or organic acids such as citric acid or amino acids such as glutamic acid.

The microorganisms may be grown under the commonly-named operating conditions of either batch or continuous culture, with or without cell recycle in either case. The culturing or fermentation is conducted under essentially anaerobic conditions with agitation of a submerged culture, at a temperature of for example 20 - 40°C, and a pH of for example 4.0 to 8.0. The preferred conditions are a temperature of about 30°C and a pH of about 5.5. It may be desirable to add certain pH regulating agents to the medium during the course of culture fermentation, such as sodium hydroxide, hydrochloric acid, or the like.

## Stage (ii)

In the second stage of the process a broth of high ethanol content is produced by fermentation. Additional carbon in amounts such that the final ethanol concentration reaches the desired level is added to the spent broth containing the bacterial biomass. The carbon may be added either stepwise or continuously as a concentrated solution, but must never exceed approximately 6 % (w/v). The options available as possible carbon sources for fermentation are described in stage (i) of the process.

The fermentation may be operated under the process conditions of so-called fed-batch or continuously, with or without cell recycle in either case. Process parameters for good production of ethanol include temperature in the range of 20 - 40°C, pH in the range of 4 to 8, mixing of the fermenting broth, and essentially anaerobic conditions. The preferred temperature is about 28 to 33°C, and the preferred pH is about 5.5. It may be necessary or desirable to add pH regulating agents as described in stage (i). Anaerobic conditions ay be maintained by bubbling a slow stream nitrogen through the broth. Occassionally additional nutrients as described in stage (i) may be added to the fermenter.

The invention will be better understood by reference to the following examples which illustrate the invention.

## Example 1

12 litres of a fermentation medium having the following composition were placed in a 14 litre fermenter vessel:

| | |
|---|---|
| Glucose | 10 % (w/v) |
| Yeast extract (Difco) | 1.0 % |
| $KH_2PO_4$ | 0,25 % |
| $NH_4Cl$ | 0.16 % |
| $MgSO_4$ | 0.1 % |
| Citric Acid | 1.0 mM |
| $CaCl_2 \cdot 2H_2O$ | 100 μM |
| $FeCl_3 \cdot 6H_2O$ | 90 μM |
| $MnCl_2 \cdot 4H_2O$ | 50 μM |
| $ZnSO_4 \cdot 7H_2O$ | 25 μM |
| $CoCl_2 \cdot 6H_2O$ | 10 μM |
| $CuCl_2 \cdot 2H_2O$ | 5 μM |
| $H_3BO_3$ | 5 μM |
| $MoO_3$ | 10 μM |
| Biotin | 1.0 mg/L |
| Ca Pantothenate | 1.5 mg/L |

50 ml of seed culture of Zymomonas mobilis ATCC 29191 grown in a medium of the above composition was added to the above fermentation medium. Cultivation was carried out at a temperature of 30°C with a nitrogen flow into the fermenter and with stirring at a rate of 300 rpm. The pH was maintained at 5.5 with 8N KOH.

After growth had terminated, the bacterial cells were collected by centrifugation and resuspended in fresh medium of the above composition to give a bacterial cell concentration of 20 g/L.

1200 ml of the concentrated bacterial cell suspension were placed in a 2 litre fermenter vessel. Stage (ii) of the process was initiated by the addition of 32 ml of a 75 % glucose solution to the fermenter. A further 450 ml of 75 % glucose was then pumped into the fermenter vessel over the course of 2 hours. Utilization of the sugar was complete after 2.5 hours giving an ethanol concentration of 10.1 %.

Stage (ii) of the process was repeated a further four times using whe bacterial cells generated by a single stage (i) of the process. After each passage through stage (ii), the bacterial cells were collected by centrifugation and resuspended in 1200 ml of fresh medium of the above composition. Glucose was added in the manner described above for the first passage of the cells through stage (ii). In each of the four cycles utilizing the same biomass, the glucose was completely utilized after 2.5 hours with an ethanol concentration of approximately 10 % being obtained in each instance.

## Example 2

Stage (i) of the process was conducted in a 740 ml fermenter containing 320 ml of the fermentation medium described in Example 1 and stage (ii) was conducted in a 2 litre fermenter containing 1740 ml of the same medium.

The stage (i) fermenter was inoculated with 7 ml of seed culture of Zymomonas mobilis ATCC 29191 grown in a medium of the composition described in example 2; stage (ii) was inoculated with 12 ml of the same seed culture. Cultivation was carried out at 30°C with a nitrogen flow into the fermenters and with a stirring rate of 300 rpm. The pH of each fermenter was maintained at 5.5 with 2N KOH.

After growth had terminated two pumps were employed to make the system continuous. The first pump supplied sterile medium containing 12.6 % glucose (other components as in Example 2) at a flow rate of 78 ml/hr. Under these conditions, the bacterial cell concentration in the stage (i) fermenter was 4.5 g/L, the ethanol concentration was 5.3 % and the residual glucose concentration was 0.6 %. The volume of the stage (i) fermenter was maintained at 320 ml by continuously transferring culture to the stage (ii) fermenter at a flow rate of 78 ml/hr. The second pump supplied a sterile 70 % glucose solution to the stage (ii) fermenter at a flow rate of 24 ml/hr. giving a total flow in and out of the fermenter of 102 ml/hr. Under these conditions, the bacterial cell concentration in the stage (ii) fermenter was 4.4 g/L, the ethanol concentration was 9.9 % and the residual glucose concentration was 1.2 %.

## Example 3

In this example Zymomonas mobilis ATCC 29191 was cultured in two fermenters which were operated in tandem in a similar manner as described in Example 2. In this example, however, the biomass contained in the effluent from the second fermenter was retained and added back to the second fermenter. Biomass recycle to the second fermenter was achieved by continuously processing the contents through a Pellicon Ultrafilter Cassette® (Millipore Corp.) containing 6 sq. ft. of HA type Millipore filters. The culture medium used in this example was of the same composition as examples 2 and 3 except the yeast extract was 0.5 % (w/v). The constant volume of the first fermenter (stage (i)) was 1600 ml. It was fed culture medium containing glucose (131 g/L) at a constant rate of 414 ml/hr. The temperature and pH were maintained at 30°C and 5.5 respectively. The steady-state concentration of biomass in the first fermenter was 4.2 g D.W/L and the ethanol was 5.1 % (w/v).

The effluent from the first fermenter was fed continuously and directly to a second fermenter which was operated at a constant volume of 760 ml. A solution of 60 % w/v glucose was pumped into the second fermenter at a constant rate of 120 ml/hr. The effluent from the second fermenter was processed through the filtration system with the cells being returned to the second fermenter. The biomass level in the second fermenter was 48 g D.W/L and was kept relatively constant by removing biomass at a rate of about 27 ml/hr. The concentration of ethanol in the cell-free filtrate leaving the system was 10.4 % w/v and the residual sugar was 1.7 %. Operation of the two stage system at an elevated biomass level in the second fermenter as a consequence of effluent filtration and biomass recycling resulted in an increased ethanol productivity of the second fermenter (product produced per unit volume per unit time).

## Claims

1. A process for producing ethanol by fermentation, which comprises culturing a microorganism, capable of producing ethanol, and which is a member of the genus Zymomonas in two stages comprising (i) producing a bacterial cell suspension together with ethanol in an ethanol concentration range that does not substantially inhibit production of the bacterial cells in a medium containing a source of nitrogen and a source of carbon, and (ii) producing ethanol in the absence of substantial bacterial cell production by the addition of fermentable sugar to the bacterial cell suspension produced in (i), wherein the sugar concentration in stage (ii) does not exceed 6 % w/v and wherein either

a) the bacterial cells are collected and resuspended in further medium between stages (i) and (ii) or

b) the two stages of the process are conducted in two fermenters with the bacterial cells produced by continuous culture in the first fermenter being continuously transferred to the second of the fermenters for stage (ii).

2. A process as claimed in claim 1 wherein the bacterial cells are produced in a mineral salt medium containing $NH_4^+$ ions as the sole source of nitrogen or in such a medium supplemented with an organic source of nitrogen.

3. A process as claimed in claim 1 or claim 2, wherein bacterial cell and ethanol production are conducted under anaerobic conditions within a pH range of 4 to 8 and a temperature range of 20 - 40°C.

4. A process claimed in any one of the preceding claims wherein the carbon source for bacterial cell and ethanol production is glucose, fructoce or sucrose, any mixture of these sugars or a polysaccharide hydrolysate containing one or more of these sugars.

5. The process of any one of the preceding claims, wherein the two stages of the process are conducted in the same fermenter by continuos addition of the fermentable sugar after termination of bacterial cell production.

6. A process as claimed in any one of claims 1 to 4 wherein the two stages of the process are conducted in the same fermenter by intermittent addition of the fermentable sugar after termination of bacterial cell production.

7. The process of claim 5 or 6, wherein the bacterial cells are collected at the end of the second stage of the process and resuspended in a fermentable sugar solution and the bacterial cells are returned to stage (ii).

8. The process of any one of claims 1 to 4, wherein the two stages of the process are conducted in two fermenters with the second fermenter being a constant volume fermenter which permits the complete utilization of a continuos feed of the fermentable sugar required for the second stage of the process.

9. The process of claim 8, wherein a portion of the bacterial cells from the second fermenter are removed from the effluent by continuous filtration or sedimentation and returned to stage (ii).


**Patentansprüche**

1. Verfahren zur Herstellung von Ethanol durch Fermentation, das umfaßt die Kultivierung eines Mikroorganismus, der Ethanol bilden kann und der ein Vertreter des Genus <u>Zymomonas</u> ist, in zwei Stufen, umfassend

i) die Herstellung einer Bakterienzellensuspension zusammen mit Ethanol in einem Ethanol-Konzentrationsbereich, der die Bildung der Bakterienzellen in einem eine Stickstoffquelle und eine Kohlenstoffquelle enthaltenden Medium nicht wesentlich hemmt, und

ii) die Herstellung von Ethanol in Abwesenheit einer wesentlichen Bakterienzellenbildung durch Zugabe eines fermentierbaren Zuckers zu der in (i) gebildeten Bakterienzellensuspension, wobei die Zuckerkonzentration in der Stufe (ii) 6 Gew. /Vol % nicht übersteigt und worin entweder

a) die Bakterienzellen gesammelt und erneut suspendiert werden in einem weiteren Medium zwischen den Stufen (i) und (ii) oder

b) die beiden Stufen des Verfahrens in zwei Fermentern durchgeführt werden, wobei die durch kontinuierliche Kultivierung in dem ersten Fermenter gebildeten Bakterienzellen zur Durchführung der Stufe (ii) kontinuierlich in den zweiten Fermenter überführt werden.

2. Verfahren nach Anspruch 1, worin die Bakterienzellen in einem Mineralsalzmedium, das $NH_4^+$-Ionen als einzige Stickstoffquelle enthält, oder in einem solchen Medium, das durch eine organische Stickstoffquelle ergänzt ist, gebildet werden.

3. Verfahren nach Anspruch 1 oder 2, worin die Bakterienzellen- und Ethanol-Bildung unter anaeroben Bedingungen innerhalb eines pH-Wertbereiches von 4 bis 8 und innerhalb eines Temperaturbereiches von 20 bis 40°C durchgeführt werden.

4. Verfahren nach einem der vorhergehenden Ansprüche, worin die Kohlenstoffquelle für die Bakterienzellen- und Ethanol-Bildung Glucose, Fructose oder Saccharose, irgendein Gemisch dieser Zucker oder ein Polysaccharid-Hydrolysat, das einen oder mehrere dieser Zucker enthält, ist.

5. Verfahren nach einem der vorhergehenden Ansprüche, worin die beiden Stufen des Verfahrens in dem gleichen Fermenter durchgeführt werden durch kontinuierliche Zugabe des fermentierbaren Zuckers nach Beendigung der Bakterienzellen-Bildung.

6. Verfahren nach einem der Ansprüche 1 bis 4, worin die beiden Stufen des Verfahrens in dem gleichen Fermenter durchgeführt werden durch intermittierende Zugabe des fermentierbaren Zuckers nach Beendigung der Bakterienzellen-Bildung.

7. Verfahren nach Anspruch 5 oder 6, worin die Bakterienzellen am Ende der zweiten Stufe des Verfahrens gesammelt und in einer fermentierbaren Zuckerlösung erneut suspendiert werden und die Bakterienzellen in die Stufe (ii) zurückgeführt werden.

8. Verfahren nach einem der Ansprüche 1 bis 4, worin die beiden Stufen des Verfahrens in zwei Fermentern durchgeführt werden, wobei der zweite Fermenter ein Fermenter mit konstantem Volumen ist, der die vollständige Ausnutzung der kontinuierlichen Beschickung aus dem fermentierbaren Zucker, der für die zweite Stufe des Verfahrens erforderlich ist, erlaubt.

9. Verfahren nach Anspruch 8, worin ein Teil der Bakterienzellen aus dem zweiten Fermenter durch kontinuierliche Filtration oder Sedimentation aus dem Abstrom entfernt und in die Stufe (ii) zurückgeführt wird.


**Revendications**

1. Procédé pour la production d'éthanol par fermentation, dans lequel on cultive un microorganisme capable de produire de l'éthanol, et qui est un membre du genre <u>Zymomonas</u>, compor-

tant deux étapes:

(1) la production d'une suspension de cellules bactériennes et de l'éthanol dans une gamme de concentration d'éthanol qui n'inhibe pratiquement pas la production des cellules bactériennes, dans un milieu contenant une source d'azote et une source de carbone, et,

(2) la production d'éthanol en l'absence de production sensible de cellules bactériennes par l'addition de sucre fermentescible à la suspension de cellules bactériennes produite en (1), dans lequel la concentration en sucre dans l'étape 2 ne dépasse pas 6 % (en poids par volume) et dans lequel soit

a) les cellules bactériennes sont recueillies et remises en suspension dans un autre milieu entre les étapes (1) et (2), soit

b) les deux étapes du procédé sont réalisées dans deux fermenteurs, les cellules bactériennes produites par culture continue dans le premier fermenteur étant transférées en continu dans le second des deux fermenteurs pour l'étape (2).

2. Procédé selon la revendication 1, dans lequel les cellules bactériennes sont produites dans un milieu de sels minéraux contenant des ions $NH^+_4$ en tant que seule source d'azote, ou dans un tel milieu auquel est ajoutée une source organique d'azote.

3. Procédé selon la revendication 1 ou la revendication 2, dans lequel la production de cellules bactériennes et d'éthanol est réalisée dans des conditions anaérobies avec un pH compris entre 4 et 8 et une gamme de température de 20 à 40°C.

4. Procédé selon l'une des revendications précédentes, dans lequel la source de carbone pour la production de cellules bactériennes et d'éthanol, est le glucose, le fructose, ou le saccharose, tout mélange de ces sucres ou un hydrolysat de polysaccharide contenant un ou plusieurs de ces sucres.

5. Procédé selon l'une des revendications précédentes, dans lequel les deux étapes du procédé sont réalisées dans le même fermenteur par addition continue du sucre fermentescible, une fois terminée la production des cellules bactériennes.

6. Procédé selon l'une des revendications 1 à 4, dans lequel les deux étapes du procédé sont réalisées dans le même fermenteur par addition intermittente du sucre fermentescible une fois terminée la production des cellules bactériennes.

7. Procédé selon la revendication 5 ou la revendication 6, dans lequel les cellules bactériennes sont recueillies à la fin de la seconde étape du procédé et replacées en suspension dans une solution de sucre fermentescible, et les cellules bactériennes sont recyclées à l'étape (2).

8. Procédé selon l'une des revendications 1 à 4, dans lequel les deux étapes du procédé sont réalisées dans deux fermenteurs, le second fermenteur étant un fermenteur à volume constant qui permet l'utilisation d'une alimentation continue en le sucre fermentescible nécessaire pour la seconde étape du procédé.

9. Procédé selon la revendication 8, dans lequel une partie des cellules bactériennes du second fermenteur est retirée de l'effluent par filtration ou sédimentation continue, et recyclée vers l'étape (2).